# EUROPEAN PATENT APPLICATION

(11) **EP 4 691 537 A1**
(43) Date of publication of application: **11.02.2026**
(21) Application number: 24780120.2
(22) Date of filing: 25.03.2024
(51) Int. Cl.: A61M 25/09, A61M 25/00, A61M 25/092

(54) **GUIDE WIRE AND CATHETER**

(30) Priority: 28.03.2023 JP 2023051294; 06.03.2024 JP 2024033836
(71) Applicant: SB-Kawasumi Laboratories, Inc., Kawasaki-shi, Kanagawa 210-8602 (JP); Sumitomo Bakelite Co., Ltd., Shinagawa-ku Tokyo 140-0002 (JP)
(72) Inventor: FUJITA, Yasuhiro, Kawasaki-shi Kanagawa 2108602 (JP)
(74) Representative: Müller-Boré & Partner Patentanwälte PartG mbB
(86) International application number: PCT/JP2024/011574
(87) International publication number: WO 2024/204002

(57) **Abstract**

A main body portion 10 includes a plurality of segments 11 and a linear member 20. The plurality of segments 11 are continuously provided in a longitudinal direction of the main body portion 10, and are configured to be separated from or closer to each other in the longitudinal direction. The linear member 20 is inserted into at least a portion of the plurality of segments 11. The linear member 20 is connected to an operation portion 30 in one end. In addition, the linear member 20 is connected to a connection segment 11a which is one of the segments 11 in the other end opposite to the one end. Since the tension of the linear member 20 extending between the connection segment 11a and the operation portion 30 is increased, the distance between the two segments 11 disposed adjacent to each other on the proximal end side with respect to the connection segment 11a is decreased, or the pressure contact force applied to the two segments 11 from each other is increased.

## Description

### Technical Field

The present invention relates to a guide wire and a catheter.

### Background Art

Some guide wires or catheters can change bending rigidity by performing an operation. With regard to this type of technology, Patent Document 1 discloses a guide wire that changes bending rigidity by rotating coils (5 and 6) via an operation member (4). Specifically, when the operation member (4) is rotationally operated in a predetermined direction, the coil (6) on a proximal end side is twisted, so that an outer diameter is decreased to increase rigidity. In addition, the coil (5) on a distal end side is pushed by the coil (6) on the proximal end side, and contracts in an axial direction to increase the rigidity. As a result, the guide wire has the increased rigidity as a whole. The outer diameter is increased as the coil (6) on the proximal end side contracts.

On the other hand, when the operation member (4) is rotationally operated in a direction opposite to the predetermined direction as described above, the coil (6) on the proximal end side contracts in the axial direction while the outer diameter of the coil (6) increases, and the bending rigidity of the coil (6) on the proximal end side decreases. In addition, the coil (5) on the distal end side expands in the axial direction by the coil (6) on the proximal end side, and the bending rigidity decreases. That is, the guide wire has the decreased bending rigidity as a whole.

### Prior Art Document

### Patent Document

[Patent Document 1] JP-A-2016-174645

### Summary of Invention

### Technical Problem

However, as described above, in the guide wire in Patent Document 1, the outer diameter of the coils (5 and 6) increases when the bending rigidity is changed. Consequently, the outer diameter of a portion of the guide wire increases. When the guide wire has a large diameter, there are problems as follows. The guide wire interferes with a vessel wall, and a blood vessel or the like is likely to be damaged when the guide wire passes through a body lumen such as the blood vessel. Moreover, the guide wire has difficulties in entering the vessel wall. In addition, there is a problem in that it is difficult to insert a catheter when the catheter is inserted into the body lumen along an outer surface of the guide wire.

The present invention has been made in view of the above-described problems, and an object of the present invention is to provide a guide wire or a catheter in which an increase in an outer diameter is suppressed to increase bending rigidity.

### Solution to Problem

According to an aspect of the present invention, there is provided a guide wire including a main body portion elongated and inserted into a body lumen, and an operation portion formed in a proximal end of the main body portion. The main body portion includes a plurality of segments continuously provided in a longitudinal direction of the main body portion and configured to be separated from or closer to each other in the longitudinal direction, and a linear member inserted into at least a portion of the plurality of segments. The linear member is connected to the operation portion in one end, and is connected to a connection segment which is one of the segments in the other end. A distance between two of the segments disposed on a proximal end side with respect to the connection segment and adjacent to each other is decreased or a pressure contact force applied to the two segments from each other is increased by increasing tension of the linear member extending between the connection segment and the operation portion.

According to an aspect of the present invention, there is provided a catheter including a main body portion elongated, inserted into a body lumen, and having a lumen, and an operation portion formed in a proximal end of the main body portion. The main body portion includes a plurality of segments continuously provided in a longitudinal direction of the main body portion and configured to be separated from or closer to each other in the longitudinal direction, and a linear member inserted into at least a portion of the plurality of segments. The linear member is connected to the operation portion in one end, and is connected to a connection segment which is one of the segments in the other end. A distance between two of the segments disposed on a proximal end side with respect to the connection segment and adjacent to each other is decreased or a pressure contact force applied to the two segments from each other is increased by increasing tension of the linear member extending between the connection segment and the operation portion.

Two segments are less likely to be bent and have increased rigidity by decreasing a distance between the segments or by increasing a pressure contact force between the segments. In this manner, bending rigidity of at least a partial length region of the guide wire or the catheter can be increased.

### Advantageous Effects of Invention

According to the guide wire or the catheter of the present invention, the bending rigidity of the guide wire or the catheter is increased by decreasing the distance between the segments or by increasing the pressure contact force between the segments. Therefore, the outer diameter of the guide wire or the catheter is rarely increased to increase the bending rigidity.

### Brief Description of Drawings

FIG. 1A is a schematic view showing an example of a guide wire according to a first embodiment of the present invention. FIG. 1B is a schematic view of the guide wire according to the first embodiment, and shows a linear member disposed inside a main body portion. FIG. 1C is a schematic view when a predetermined distance is provided between segments in the guide wire according to the first embodiment.
FIG. 2A is a perspective view of a segment in a guide wire according to the first embodiment. FIG. 2B is a plan view when the segment according to the first embodiment is viewed from a distal end side.
FIG. 3A is a schematic view showing a plurality of segments including a first segment of the guide wire according to the first embodiment. FIG. 3B is a schematic view for describing bending of the plurality of segments shown in FIG. 3A. FIG. 3C is a schematic view showing a plurality of segments including a second segment of the guide wire according to the first embodiment. FIG. 3D is a schematic view for describing bending of the plurality of segments shown in FIG. 3C. A second linear member is omitted in the drawings in FIGS. 3A to 3D.
FIG. 4A is a schematic view showing an operation portion. A portion of the main body portion is omitted in the drawing. FIG. 4B is an exploded view of the operation portion.
FIGS. 5A to 5C are schematic views showing a portion of a main body portion according to a modification example.
FIG. 6A is a schematic view showing an example of a catheter according to a second embodiment of the present invention. FIG. 6B is a schematic view of the catheter, and shows a linear member disposed inside the main body portion. FIG. 6C is a schematic view when a predetermined distance is provided between segments in the catheter.
FIG. 7 is a schematic cross-sectional view when a distal end portion of the catheter according to the second embodiment is taken along an axial direction of the catheter.
FIG. 8A is an enlarged cross-sectional view of an example of the catheter according to the second embodiment. FIG. 8B is a schematic cross-sectional view of the catheter for describing a pulling mechanism.
FIGS. 9A and 9B are enlarged cross-sectional views of another example of the catheter according to the second embodiment.
FIG. 10A is a plan view when a segment according to the second embodiment is viewed from the distal end side. FIG. 10B is a plan view when the segment is viewed from the distal end side in a case where the catheter has an operation wire.

### Description of Embodiments

Various components of a guide wire or a catheter of the present invention do not need to be individually independent, and the present invention allows that a plurality of components are formed as one member, that one component is formed of a plurality of members, that one component is a portion of another component, that a portion of one component and a portion of another component overlap each other, and the like.

In addition, a method of using the guide wire or the catheter according to the present invention will be described by using a plurality of steps described in order, but the order of the description does not limit the order or a timing of performing the plurality of steps. Therefore, when the method of using the guide wire or the catheter according to the present invention is performed, the order of the plurality of steps can be changed within a range having no hindrance in terms of content, and some or all of timings of performing the plurality of steps may overlap with each other.

Hereinafter, embodiments of the present invention will be described with reference to the drawings. In addition, in each drawing, common reference numerals will be assigned to corresponding components, and repeated description will be omitted as appropriate.

In addition, a plane referred to herein in the present invention means a shape that is physically formed by targeting a plane, and as a matter of course, it is not necessary that the plane is a geometrically perfect plane.

### <First Embodiment>

### (Guide Wire)

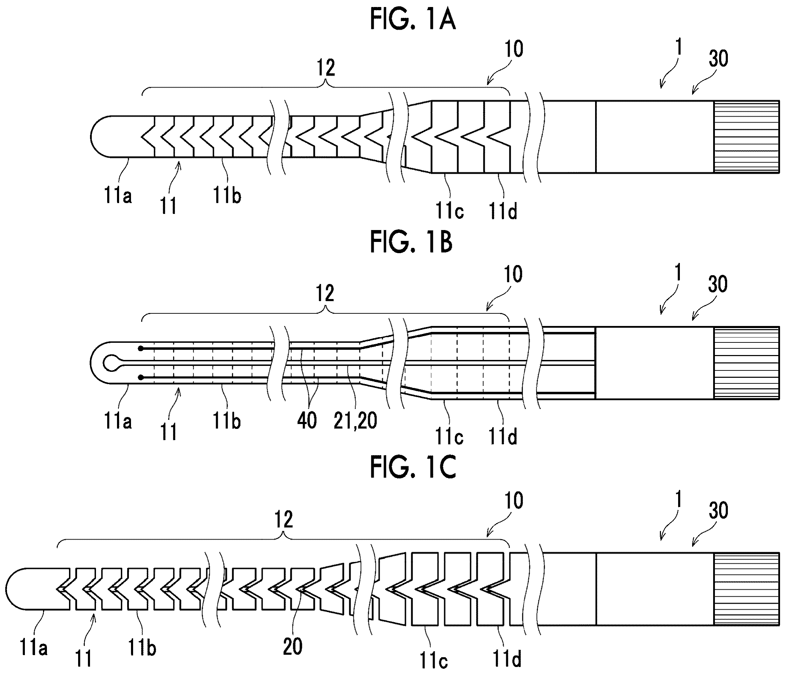

FIG. 1A is a schematic view showing an example of a guide wire 1 according to a first embodiment of the present invention.

First, an outline of the guide wire of the present embodiment will be described.

The guide wire 1 includes a main body portion 10 and an operation portion 30. The main body portion 10 is elongated, and is inserted into a body lumen. The operation portion 30 is formed in a proximal end of the main body portion 10. The main body portion 10 includes a plurality of segments 11 and a linear member (tension wire 20). The plurality of segments 11 are continuously provided in a longitudinal direction of the main body portion 10. In addition, the plurality of segments 11 can be separated from or closer to each other in the longitudinal direction of the main body portion 10. The tension wire 20 is inserted into at least a portion of the plurality of segments 11. The tension wire 20 is connected to the operation portion 30 in one end. In addition, the tension wire 20 is connected to a connection segment 11a which is one of the segments 11 in the other end opposite to the one end.

Next, the guide wire 1 of the present embodiment will be described in detail.

The guide wire 1 is a member inserted into the body lumen in a living body, is an elongated member as a whole, and includes a so-called stylet. The guide wire 1 may be used together with a catheter, and in particular, in order to allow a distal end of the catheter to enter an affected site, the guide wire 1 is inserted into a blood vessel or the like before the catheter enters the affected site. Specifically, the guide wire 1 is inserted into a desired branch destination at a branch location where the blood vessel is branched into a plurality of branch destinations.

Hereinafter, in the guide wire 1, and the main body portion 10, the tension wire 20, and the operation portion 30 to be described later, one end on a side that enters the body lumen in advance when the guide wire 1 is inserted into the body lumen will be referred to as a distal end, and one end side thereof will be referred to as a distal end side. In addition, in the guide wire 1, the main body portion 10, the tension wire 20, and the operation portion 30, one end on a side opposite to the distal end will be referred to as a proximal end, and the one end side will be referred to as a proximal end side.

The main body portion 10 is a portion inserted into the body lumen such as the blood vessel. It is not necessary to insert the whole main body portion 10 into the body lumen, and at least a portion of the main body portion 10 on the distal end side is inserted into the body lumen. An outer diameter of the main body portion 10 is smaller than an inner diameter of the body lumen. In addition, the outer diameter of the main body portion 10 is smaller than an inner diameter of a catheter (not shown) inserted into the body lumen along an outer surface of the guide wire 1 by using a technique called an over-the-wire. The outer diameter of the main body portion 10 in a partial length region on the distal end side in the present embodiment is smaller than the outer diameter in a partial length region on the proximal end side with respect to the partial length region.

An exterior forming a surface of the main body portion 10 is divided into a plurality of portions in the longitudinal direction of the main body portion 10. In other words, the exterior of the main body portion 10 includes a plurality of segments 11 aligned to overlap each other in the longitudinal direction. In particular, at least partial exterior on the distal end side of the main body portion 10 which is inserted into the body lumen includes the plurality of segments 11. Hereinafter, the longitudinal direction of the main body portion 10 may be simply referred to as the longitudinal direction or an axial direction.

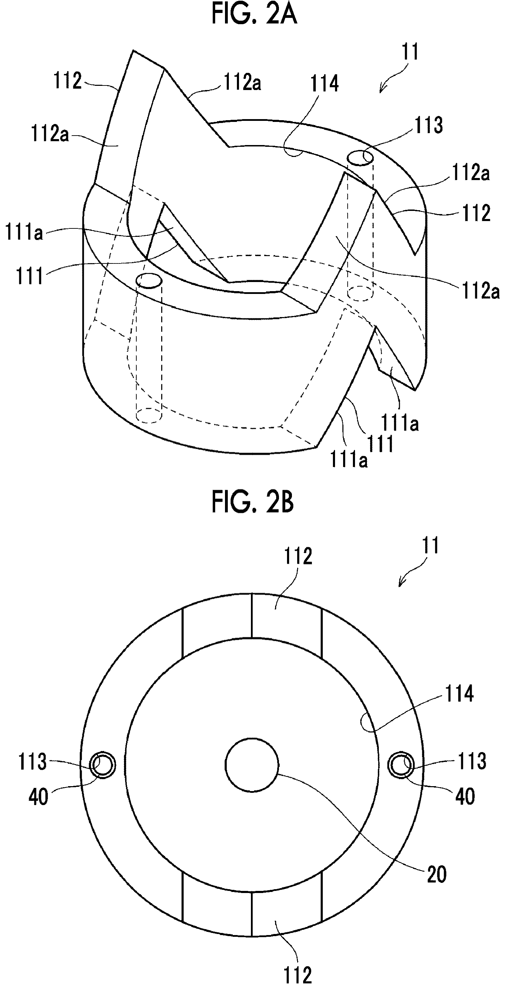

As shown in FIG. 2A, a shape of the segment 11 is an approximately cylindrical shape. The shape of the segment 11 may be a polygonal columnar shape in which a through-hole 114 (to be described later) is formed. In addition, the shape of the segment 11 may be a shape tapered toward the distal end as a whole.

The segment 11 is provided with the through-hole 114 that penetrates along the axial direction of the main body portion 10. A penetration direction of the through-hole 114 may be referred to as the axial direction of the segment 11 or simply the axial direction. A tension wire 20 (refer to FIG. 1B, to be described later) is inserted into the through-hole 114. Preferably, the tension wire 20 is loosely inserted into the through-hole 114. That is, the through-hole 114 has a shape and a dimension which are larger than a shape and a dimension of a cross section of the tension wire 20. As shown in FIG. 2A, a connection segment 11a (to be described later) may or may not have the through-hole 114. The connection segment 11a may be a solid member, may be a member internally having a hollow portion and having a closed distal end, or may be a cylindrical member provided with the through-hole 114, as in the other segments 11.

The description that the plurality of segments 11 can be separated from or closer to each other in the longitudinal direction of the main body portion 10 means that two segments 11 adjacent to each other in the longitudinal direction can decrease or increase a distance between the two segments 11 in the longitudinal direction. Specifically, in the present embodiment, the segment 11 moves or slides on the tension wire 20 in the longitudinal direction of the main body portion 10. In this manner, the distance between the segments 11 adjacent to each other in the longitudinal direction of the main body portion 10 can be changed.

Preferably, the two adjacent segments 11 are separated from each other. It is more preferable that the plurality of segments 11 are separated from each other. Here, the description that two or more of the segments 11 are separated from each other means that the segments 11 move or slide on the tension wire 20 independently of each other. That is, the above description does not include a case where the segments 11 are indirectly connected to each other via another member such as a hinge as will be described later.

Instead of the present embodiment, the segments 11 may be connected by a member such as a hinge that allows the distance or the pressure contact force between the segments 11 to be changed. Alternatively, the segments 11 may be connected to each other by a thread-like member.

The tension wire 20 is a linear member inserted into the segment 11, and is a member for adjusting bending rigidity of the main body portion 10. Specifically, the tension wire 20 is a member for pulling the connection segment 11a to the proximal end side. The tension wire 20 in the present embodiment is a metal wire. Instead of the present embodiment, the tension wire 20 may be formed of a resin or other materials. When the tension wire 20 is formed of the resin wire, it is preferable to use a high-tensile resin. It is preferable that the tension wire 20 is a member having low stretchability in the axial direction. The tension wire 20 is inserted into the through-holes 114 of at least two or more segments 11. It is preferable that the tension wire 20 is inserted into all of the segments 11 other than the connection segment 11a (to be described later). The segment 11 into which the tension wire 20 is inserted can move or slide on the tension wire 20 in the axial direction.

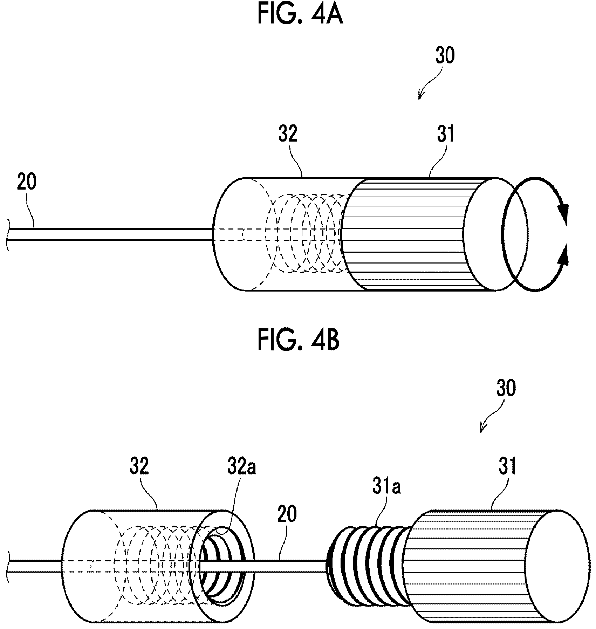

As shown in FIG. 4B, a proximal end portion of the tension wire 20 is connected to the operation portion 30. Specifically, the tension wire 20 is fixed to a twisted portion 31 (to be described later). The tension wire 20 and the operation portion 30 are connected to each other by embedding, adhesion, brazing, welding, or any other methods. In addition, the tension wire 20 and the operation portion 30 may be connected to each other via another member.

As shown in FIG. 1B, the distal end portion of the tension wire 20 is connected to the connection segment 11a which is one of the segments 11. The tension wire 20 is connected to the connection segment 11a by embedding, adhesion, brazing, welding, or any other methods. In the present embodiment, the connection segment 11a is a segment 11 disposed at a most distal end position in the main body portion 10 in the plurality of segments 11. In other words, the connection segment 11a is a segment 11 forming a most distal end portion of the main body portion 10. Instead of the present embodiment, the segment 11 disposed in an intermediate portion of the main body portion 10 in the longitudinal direction may be set as the connection segment 11a, or the segment 11 disposed on the proximal end side may be set as the connection segment 11a.

The operation portion 30 is a portion in the guide wire 1 for adjusting tension of the tension wire 20. In addition, a bending operation of the main body portion 10 may be performed by the operation portion 30 via an operation wire 40 (to be described later).

As shown in FIG. 4A, the operation portion 30 in the present embodiment includes a twisted portion 31 and a female screw portion 32. As shown in FIG. 4B, a male screw 31a is provided on the distal end side of the twisted portion 31, and a female screw 32a is provided on the proximal end side of the female screw portion 32. The female screw 32a has a shape and a dimension which correspond to the male screw 31a. The female screw portion 32 and the twisted portion 31 are connected to each other by fastening the male screw 31a to the female screw 32a.

The tension applied to the tension wire 20 is increased or decreased by adjusting a fastening degree between the female screw portion 32 and the twisted portion 31. Specifically, when the twisted portion 31 is rotationally operated in any direction of an arrow shown in FIG. 4A, the twisted portion 31 moves to the proximal end side, and is separated from the female screw portion 32. In this manner, the tension wire 20 is pulled to the proximal end side, and the tension applied to the crossing portion 21 (to be described later) increases. In some cases, a length of a crossing portion 21 (to be described later) may be decreased by pulling the tension wire 20. The tension wire 20 may be twisted by fastening the female screw 32a and the male screw 31a. Alternatively, the tension wire 20 and the twisted portion 31 may be connected to each other such that twisting of the tension wire 20 is eliminated when the male screw 31a and the female screw 32a are fastened.

On the other hand, when the twisted portion 31 is rotationally operated in a direction opposite to the direction of the arrow shown in FIG. 4A, the twisted portion 31 moves closer to the female screw portion 32. In this manner, the tension wire 20 is loosened, and the tension applied to the crossing portion 21 decreases. The length of the crossing portion 21 may be increased by loosening the tension wire 20.

A shape of the operation portion 30 is not limited to a shape of the present embodiment, and the operation portion 30 may have any shape as long as the operation portion 30 has a mechanism for adjusting the tension of the tension wire 20. For example, the operation portion 30 may have a fixing portion for pulling a portion of the tension wire 20 and maintaining a pulled state by locking or the like. More specifically, the operation portion 30 may have a mechanism for winding the tension wire 20.

In addition, the operation portion 30 of the present embodiment may have a latch structure to maintain the fastening degree between the twisted portion 31 and the female screw portion 32. For example, a structure in which the male screw 31a and the female screw 32a are locked to each other may be provided, and the male screw 31a may be locked to be prevented from being unintentionally rotated with respect to the female screw 32a by the twisted tension wire 20 or the tension wire 20 to which high tension is applied.

The adjustment of the bending rigidity of the main body portion 10 will be described with reference to FIGS. 1A to 1C.

Since the tension of the tension wire 20 extending between the connection segment 11a and the operation portion 30 increases, the distance between the two segments 11 disposed adjacent to each other on the proximal end side with respect to the connection segment 11a decreases, or the pressure contact force applied to the two segments 11 from each other increases. In this manner, the bending rigidity of the main body portion 10 is adjusted and increased.

Specifically, the tension wire 20 shown in FIG. 1B is pulled to the proximal end side by operating the operation portion 30 as described above. When the tension wire 20 is pulled to the proximal end side, the tension applied to the tension wire 20 increases. More specifically, the tension of a partial length region (crossing portion 21) inserted into the plurality of segments 11 in the tension wire 20 increases. The crossing portion 21 is a partial length region of the tension wire 20 extending from the segment on the most proximal end side (proximal end segment 11d) throughout the connection segment 11a.

In a state before the operation (also referred to as an initial state), in some cases, a predetermined distance may be provided between the adjacent segments 11 as shown in FIG. 1C. In other words, in some cases, the length of the crossing portion 21 may be larger than a total length of the plurality of segments 11 when the plurality of segments 11 are aligned in contact with each other in the axial direction. In this case, when the tension wire 20 is pulled to the proximal end side to apply the tension to the tension wire 20, the length of the crossing portion 21 decreases. The reason is that a portion on the proximal end side in the partial length region which is the crossing portion 21 in the initial state is pulled to the proximal end side and is disposed on the proximal end side with respect to the proximal end segment 11d. When the length of the crossing portion 21 decreases, a distance decreases between two adjacent segments 11 (hereinafter, also referred to as an inter-segment distance) in the plurality of segments 11 (hereinafter, the plurality of segments 11 will be collectively referred to as or the partial length region in which the plurality of segments 11 are disposed will be referred to as a flexible portion 12) into which the crossing portion 21 is inserted. In this manner, the movement of the one segment 11 is restricted such that the axial direction of the one segment 11 intersects with the axial direction of the other segment 11 (adjacent segment) adjacent to the one segment 11. Hereinafter, the description that one segment 11 moves with respect to the adjacent segment in this way may mean that one segment 11 is bent with respect to the adjacent segment. Specifically, when the inter-segment distance is small, and when one segment 11 attempts to be bent with respect to the adjacent segment, a portion of the one segment 11 comes into contact with the adjacent segment. In this manner, bending of the one segment 11 is locked by the adjacent segment. That is, when the inter-segment distance is small, a bending angle (to be described later) of the one segment 11 with respect to the adjacent segment decreases. Since the bending angle between the adjacent segments 11 is small, flexibility of the whole flexible portion 12 decreases. In other words, the whole flexible portion 12 has increased bending rigidity.

On the other hand, as shown in FIG. 1A, in some cases, the adjacent segments 11 may be in contact with or may be in pressure contact with each other in the initial state. In other words, in some cases, the length of the crossing portion 21 is smaller than the total length of the plurality of segments 11 when the plurality of segments 11 are aligned in contact with each other in the axial direction. In this case, when the tension wire 20 is pulled to the proximal end side to apply the tension to the tension wire 20, the pressure contact force applied to the segments 11 from each other increases. The pressure contact force described herein is stress in the axial direction which is directly or indirectly applied to side end surfaces of the adjacent segments 11. The side end surfaces of the adjacent segments 11 are in direct contact with each other, and one side end surface of the segment 11 may be in pressure contact with the other side end surface of the segment 11. Alternatively, the other member (for example, an outer layer 50 or an inner layer 60 in a second embodiment to be described later) may be interposed between the side end surfaces of the adjacent segments 11, and one segment 11 may be indirectly in pressure contact with the other segment 11 by being in pressure contact with the other member. Since the pressure contact force between the segments 11 increases, the flexible portion 12 contracts as a whole in the axial direction, and the bending rigidity increases.

In addition, in contrast to the above-described operation, the tension of the tension wire 20 extending between the connection segment 11a and the operation portion 30 may be decreased such that the distance between the two segments 11 disposed adjacent to each other on the proximal end side with respect to the connection segment 11a increases or the pressure contact force applied to the two segments 11 from each other decreases. In this manner, the bending rigidity of the main body portion 10 is adjusted and decreased. That is, flexibility of the main body portion 10 increases.

In this way, the distal end of the main body portion 10 can easily reach a desired position in the body lumen by adjusting the main body portion 10 to have desired bending rigidity. For example, in some cases, the bending rigidity required for the guide wire 1 may vary depending on a size of a diameter of the blood vessel. Specifically, it is necessary to use the guide wire 1 having high bending rigidity when the guide wire 1 starts to be inserted. However, in some cases, it may be necessary to use the guide wire 1 having low bending rigidity and high flexibility when the guide wire 1 is caused to enter a peripheral blood vessel. In this way, the bending rigidity of the guide wire 1 may be changed according to characteristics such as a thickness of the blood vessel.

In addition, for example, in order to cause the guide wire 1 to enter a desired branch destination, the bending rigidity of the guide wire 1 can be changed at a branch location of the blood vessel. Specifically, after the distal end of the guide wire 1 is caused to enter the branch location of the blood vessel, in some cases, the distal end of the main body portion 10 may need to be bent to cause the distal end of the guide wire 1 to enter the desired branch destination. In this case, the bending rigidity of the main body portion 10 can be decreased to increase the flexibility, and the distal end of the main body portion 10 can be bent at a desired angle corresponding to a shape of the branch location by performing the bending operation. Furthermore, in order to maintain a bent state at a desired angle and to ensure pushability of the guide wire 1, the bending rigidity of the main body portion 10 may be increased after the bending operation.

As described above, in the guide wire 1 of the present embodiment, the distance between the segments 11 is decreased or the pressure contact force between the segments 11 is increased. In this manner, the two segments 11 are less likely to be bent and have increased rigidity. In this manner, the bending rigidity of at least the partial length region of the guide wire 1 can be increased. The bending rigidity of the guide wire 1 increases since the distance between the segments 11 decreases or the pressure contact force between the segments 11 increases. Therefore, an outer diameter of the guide wire 1 is rarely increased to increase the bending rigidity.

As shown in FIG. 1C, one segment 11 in the present embodiment has an uneven engagement portion. Specifically, at least one segment 11 of the segments 11 forming the flexible portion 12 has the uneven engagement portion. Preferably, all of the segments 11 forming the flexible portion 12 have the uneven engagement portions. The uneven engagement portions engage with each other in the other adjacent segments 11.

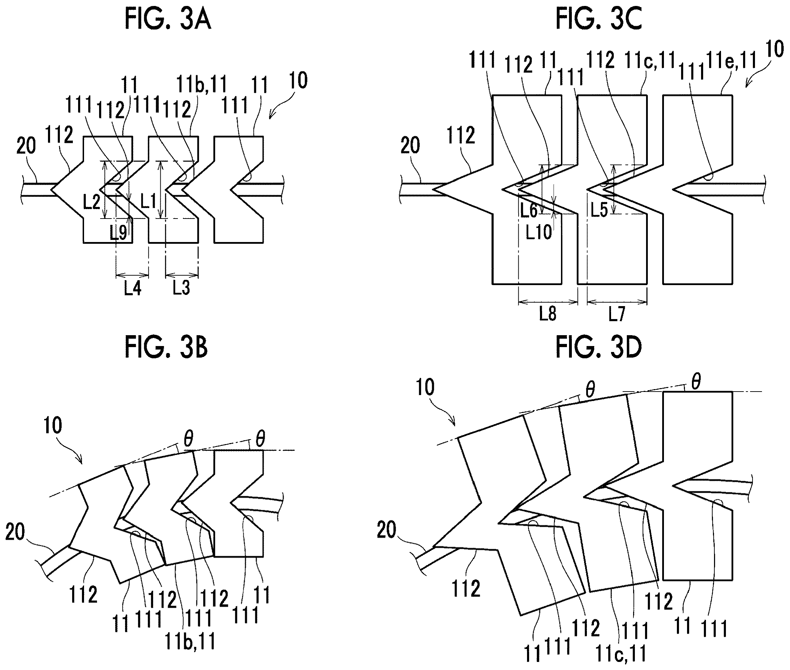

Specifically, as shown in FIG. 3A, the one segment 11 has a recess portion 111 as the uneven engagement portion in the proximal end portion. The one segment 11 refers to any segment 11 in the flexible portion 12, but for convenience of description, the one segment 11 will be described as a first segment 11b (to be described later) shown in FIG. 3A. Another segment 11 (adjacent segment 11e shown in FIG. 3A) adjacent to the first segment 11b has a protruding portion 112 as the uneven engagement portion in the distal end portion. The adjacent segment 11e in FIG. 3A is adjacent to the first segment 11b on the proximal end side (right side of the paper surface) of the first segment 11b.

The recess portion 111 in the present embodiment is formed in such a manner that a cylindrical wall portion forming the segment 11 is recessed in the axial direction. Similarly, the protruding portion 112 is formed in such a manner that a cylindrical wall portion forming the segment 11 protrudes in the axial direction. It is preferable that the shape of the recess portion 111 in the first segment 11b and the shape of the protruding portion 112 in the adjacent segment 11e correspond to each other, but an example is not limited to a case where the shape of the recess portion 111 and the shape of the protruding portion 112 are strictly the same as each other. The recess portion 111 and the protruding portion 112 may have slightly different shapes, such as having mutually different width dimensions. For example, the width of the recess portion 111 may be larger than the width of the protruding portion 112.

The first segment 11b and the adjacent segment 11e engage with each other such that the recess portion 111 of the first segment 11b and the protruding portion 112 of the adjacent segment 11e overlap each other when viewed in the circumferential direction of the main body portion 10. In this manner, the rotation of the first segment 11b in the circumferential direction with respect to the adjacent segment 11e is locked. In other words, in this manner, torsional rigidity of the main body portion 10 can increase, and a torque operability can be improved. The circumferential direction of the main body portion 10 is a direction around an axis of the main body portion 10. Hereinafter, the circumferential direction of the main body portion 10 may be simply referred to as the circumferential direction.

In the present embodiment, the segment 11 has two recess portions 111 and two protruding portions 112. Specifically, as shown in FIG. 2B, the uneven engagement portions (recess portion 111 (refer to FIG. 2A) or protruding portion 112) are disposed to face each other at an angle of 180 degrees across the through-hole 114. The segment 11 may have only one uneven engagement portion (recess portion 111 or protruding portion 112), or may have a plurality of the uneven engagement portions including two or more.

The recess portion 111 or the protruding portion 112 is formed to be recessed or to protrude toward either side in the axial directions. In the present embodiment, as shown in FIG. 3A, the recess portion 111 or the protruding portion 112 is formed to be recessed or to protrude toward the distal end. From a viewpoint of flexibility of the main body portion 10, it is preferable that the uneven engagement portion is formed to be recessed or to protrude toward the distal end, compared to a case where the uneven engagement portion is formed to be recessed or to protrude toward the proximal end. In addition, as shown in FIG. 2A, the two recess portions 111 or the two protruding portions 112 are formed to be recessed or to protrude in the same direction. Instead of the present embodiment, the recess portion 111 or the protruding portion 112 may be formed to be recessed or to protrude toward the proximal end.

In the present embodiment, the width of the protruding portion 112 and the width of the recess portion 111 are narrowed toward the distal end of the main body portion 10. The width of the protruding portion 112 or the recess portion 111 is a dimension of the protruding portion 112 or the recess portion 111 in the circumferential direction of the main body portion 10. In other words, the width of the protruding portion 112 or the recess portion 111 is a dimension along a peripheral surface of the segment 11 in the protruding portion 112 or the recess portion 111.

Specifically, as shown in FIG. 2A, the recess portion 111 has a pair of wall portions (referred to as recess portion wall portions 111a). Each of the pair of recess portion wall portions 111a is disposed obliquely with respect to the axial direction. The protruding portion 112 also has a pair of wall portions (referred to as protruding wall portions 112a). Each of the pair of protruding wall portions 112a is also disposed obliquely with respect to the axial direction. A small angle in angles formed by the axial direction and the recess portion wall portion 111a or the protruding wall portion 112a will be referred to as an inclination angle of the recess portion wall portion 111a or the protruding wall portion 112a.

As shown in FIGS. 3A to 3D, the recess portion wall portion 111a (refer to FIG. 2A) in the first segment 11b and the protruding wall portion 112a (refer to FIG. 2A) in the adjacent segment 11e are disposed to face each other. That is, the recess portion wall portion 111a in the first segment 11b and the protruding wall portion 112a in the adjacent segment 11e are disposed along each other. The recess portion wall portion 111a of the recess portion 111 and the protruding wall portion 112a of the protruding portion 112, which engage with each other, are disposed along each other. Therefore, the bending of the segments 11 adjacent to each other is appropriately controlled. For example, as shown in FIG. 3B or 3D, when the adjacent segments 11 attempt to be bent, the recess portion wall portion 111a and the protruding wall portion 112a come into contact with each other. Therefore, the bending is locked at a predetermined position. Specifically, as shown in FIG. 3B, the segments 11 may come into contact with each other on a side end surface of the segment 11 (side end surface on which the uneven engagement portion is not formed). Alternatively, as shown in FIG. 3D, the recess portion 111 of the segment 11 and the protruding portion 112 of the adjacent segment 11 may come into contact with each other.

The protruding portion 112 and the recess portion 111 in the present embodiment have a shape pointed toward the distal end. Instead of the present embodiment, a protruding end in the protruding portion 112 may be flat, or a bottom portion of the recess portion 111 may be flat.

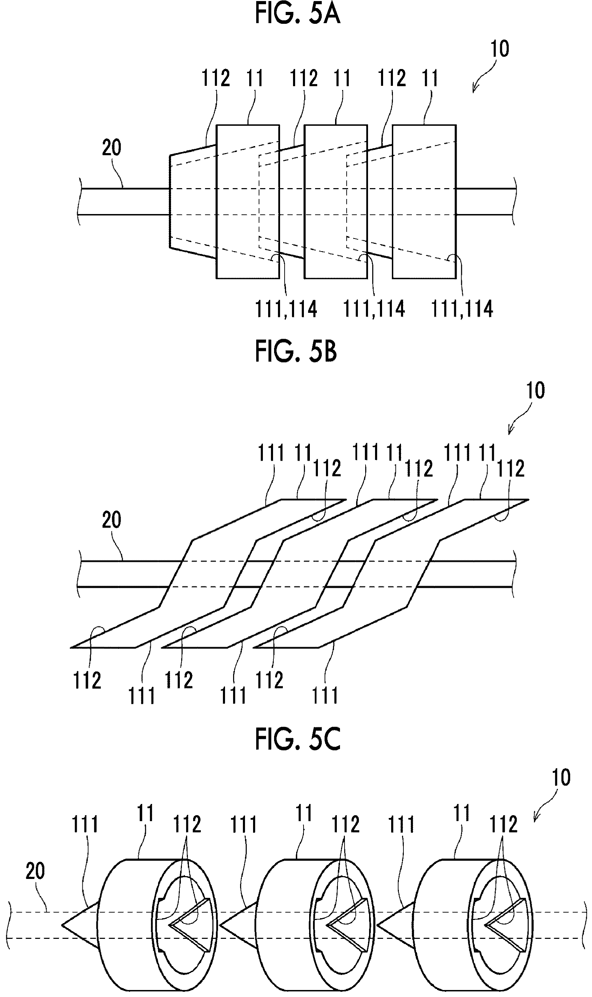

As shown in FIG. 2A, the recess portion 111 and the protruding portion 112 in the present embodiment are formed on an entire wall thickness of the segment 11, but the present invention is not limited thereto. For example, as shown in FIG. 5C, the recess portion 111 and the protruding portion 112 may be formed on an inner diameter side in a wall thickness direction of the segment 11, and may not be formed on an outer diameter side. When the protruding portion 112 is fitted into the recess portion 111 of the adjacent segment 11, the protruding portion 112 is accommodated inside the segment 11 (on the inner diameter side with respect to a peripheral wall of the segment 11). In the segment 11 having this uneven engagement portion, when the adjacent segments 11 are bent, the protruding portion 112 does not protrude outward from an outer surface of the segment 11, and interference with a catheter or a vascular wall is prevented. Alternatively, in the segment 11 having the uneven engagement portion, bending of the segments 11 adjacent to each other is restricted in the wall thickness direction of the segment 11 (front-back direction of the paper surface in FIG. 1A or FIG. 5C) in a portion where the uneven engagement portion is provided. The reason is that the protruding portion 112 interferes with the peripheral wall of the adjacent segment 11. Therefore, the main body portion 10 can be exclusively moved in a desired oscillating direction (up-down direction in the drawing in FIG. 1A or FIG. 5C). In FIG. 5C, the segments 11 are separated from each other in the drawing for convenience.

In the present embodiment, the width of the protruding portion 112 or the recess portion 111 provided in the first segment (first segment 11b) is larger than the width of the protruding portion 112 or the recess portion 111 provided in the second segment (second segment 11c). That is, the width of the recess portion 111 of the first segment 11b (dimension L1 in FIG. 3A) is larger than the width of the recess portion 111 of the second segment 11c (dimension L5 in FIG. 3C). Alternatively, the width of the protruding portion 112 of the first segment 11b (dimension L2 in FIG. 3A) is larger than the width of the protruding portion 112 of the second segment 11c (dimension L6 in FIG. 3C). The width of the protruding portion 112 or the width of the recess portion 111, which is compared here, is a width dimension on the proximal end side of the protruding portion 112 or the recess portion 111.

The first segment 11b is any segment 11 of the segments 11 forming the flexible portion 12. Alternatively, the first segment 11b may be the connection segment 11a. The second segment 11c is the segment 11 disposed on the proximal end side with respect to the first segment 11b. The second segment 11c may be the segment 11 adjacent to the proximal end side of the first segment 11b, or may be the segment 11 disposed on the proximal end side with respect to the first segment 11b without being adjacent to the first segment 11b.

The width of the uneven engagement portion (the recess portion 111 or the protruding portion 112) in the segment 11 may gradually decrease from the segment 11 disposed on the distal end side to the segment 11 disposed on the proximal end side. Alternatively, a group of the plurality of segments 11 disposed on the distal end side may have the uneven engagement portions having substantially the same width, and a group of the plurality of segments 11 disposed on the proximal end side with respect to the group of the segments 11 may have the uneven engagement portions having a smaller width.

When the width of the recess portion 111 or the protruding portion 112 is large, larger bending of the two adjacent segments 11 can be allowed, compared to a case where the width is small. In other words, when the width of the recess portion 111 or the protruding portion 112 is small, a bending angle in the bending of the two adjacent segments 11 is limited to be small, compared to a case where the width is large.

This configuration will be described with reference to FIGS. 3A and 3C. When a depth dimension of the recess portion 111 or a protruding dimension of the protruding portion 112 is the same, and when the width of the recess portion 111 or the protruding portion 112 is large, a separation distance between the adjacent recess portions 111 and the adjacent protruding portions 112 in the circumferential direction increases, compared to a case where the width is small. The reason is that the inclination angle of the recess portion wall portion 111a or the protruding wall portion 112a increases when the width is large, compared to a case where the width is small. That is, the inclination angle of the recess portion wall portion 111a or the protruding wall portion 112a in FIG. 3A is larger than the inclination angle of the recess portion wall portion 111a or the protruding wall portion 112a in FIG. 3C, and a separation distance L9 shown in FIG. 3A is larger than a separation distance L10 shown in FIG. 3C. Since the separation distance in the circumferential direction between the segments is different, when two adjacent segments 11 attempt to be bent, a distance until the recess portion 111 and the protruding portion 112 of the two segments come into contact with each other differs, and a maximum value of the bending angle differs. In addition, as the inclination angle of the recess portion wall portion 111a or the protruding wall portion 112a is smaller, the adjacent segments 11 are restricted from being bent at the inclination angle or larger. The reason is as follows. As shown in FIG. 3D, even when attempting to be bent at the inclination angle or larger, the recess portion wall portion 111a (refer to FIG. 2A) and the protruding wall portion 112a (refer to FIG. 2A) come into contact with each other, and the bending is locked.

Here, the bending angle is an angle formed by the axial directions of the two segments 11 when the two adjacent segments 11 are bent. Specifically, the bending angle is an angle represented by θ in FIGS. 3B and 3D.

The width of the recess portion 111 or the protruding portion 112 in the segment 11 disposed on the distal end side and the segment 11 disposed on the proximal end side is changed in this way. In this manner, flexibility of the distal end portion of the main body portion 10 can be increased, and flexibility of the proximal end portion of the main body portion 10 can be restricted to increase the bending rigidity.

In the present embodiment, the protruding dimension of the protruding portion 112 or the depth dimension of the recess portion 111 in the first segment 11b is smaller than the protruding dimension of the protruding portion 112 or the depth dimension of the recess portion 111 in the second segment 11c. That is, the depth dimension of the recess portion 111 in the first segment 11b (dimension L3 in FIG. 3A) is smaller than the depth dimension of the recess portion 111 in the second segment 11c (dimension L7 in FIG. 3C). In addition, the protruding dimension of the protruding portion 112 in the first segment 11b (dimension L4 in FIG. 3A) is smaller than the protruding dimension of the protruding portion 112 in the second segment 11c (dimension L8 in FIG. 3C). The protruding dimension of the protruding portion 112 or the depth dimension of the recess portion 111 is a dimension of the recess portion 111 or the protruding portion 112 in the longitudinal direction of the main body portion 10.

When the widths of the recess portion 111 or the protruding portion 112 are equal, and when the depth dimension of the recess portion 111 or the protruding dimension of the protruding portion 112 is small, the separation distance between the adjacent recess portion 111 and the adjacent protruding portion 112 in the circumferential direction increases, compared to a case where the dimension is large. In addition, when the dimension is small, the inclination angle of the recess portion wall portion 111a or the protruding wall portion 112a increases, compared to a case where the dimension is large. Therefore, when the dimension is small, the maximum value of the bending angle between the adjacent segments 11 increases, compared to a case where the dimension is large. That is, the dimension in the segment 11 on the distal end side is decreased, and the dimension in the segment 11 on the proximal end side is increased. Therefore, the flexibility of the distal end portion of the main body portion 10 can be increased, and the flexibility of the proximal end portion of the main body portion 10 can be restricted to increase the bending rigidity.

The depth dimension of the recess portion 111 or the protruding dimension of the protruding portion 112 in the segment 11 may gradually increase from the segment 11 disposed on the distal end side to the segment 11 disposed on the proximal end side. Alternatively, the dimensions of the uneven engagement portions in the group of the plurality of segments 11 disposed on the distal end side may be substantially the same dimension, and the dimensions of the uneven engagement portions in the group of the plurality of segments 11 disposed on the proximal end side with respect to the group of the segments 11 may be larger than the dimensions of the uneven engagement portions in the group of the plurality of segments 11 disposed on the distal end side.

In the present embodiment, as shown in FIGS. 3A to 3D, the dimension of the segment 11 (for example, the first segment 11b) in the longitudinal direction (axial direction) is smaller than the width dimension of the segment (first segment 11b) in a direction orthogonal to the longitudinal direction. Here, the dimension of the segment 11 in the longitudinal direction is a dimension that does not include the protruding dimension of the protruding portion 112 when the protruding portion 112 is provided. That is, the segment 11 is formed to include a cylindrical wall portion and the protruding portion 112 that protrudes from the cylindrical wall portion in the axial direction, but the dimension of the segment 11 in the longitudinal direction refers to the dimension of only the cylindrical wall portion. In addition, the width dimension of the segment 11 is a dimension of the segment 11 in the direction orthogonal to the axial direction when the main body portion 10 is viewed from a side surface side of the main body portion 10 (for example, when the main body portion 10 is viewed as in FIGS. 1 or 3A to 3D). When a cross section of the segment 11 has a circular shape, the width dimension of the segment 11 is also the outer diameter of the segment 11. That is, the main body portion 10 in the present embodiment is finely divided in the axial direction. Since the main body portion 10 is finely divided in the axial direction, the main body portion 10 can be bent at the divided location. Therefore, the main body portion 10 can be flexibly bent, and can obtain high flexibility, compared to a case where the dimension of the segment 11 in the axial direction is larger than the width dimension of the segment 11.

Preferably, the dimension in the axial direction is smaller than the width dimension in at least the plurality of segments 11 on the distal end side which form the flexible portion 12, without being limited to the first segment 11b. More preferably, the dimension in the axial direction is smaller than the width dimension in all of the segments 11 forming the flexible portion 12.

The dimension of the first segment 11b in the longitudinal direction (axial direction) is smaller than the dimension of the second segment 11c in the longitudinal direction (axial direction). In the flexible portion 12, as the segment 11 is disposed toward the distal end side, the dimension of the segment 11 in the axial direction may gradually increase. Alternatively, the dimension in the axial direction in the group of the plurality of segments 11 disposed on the distal end side may be smaller than the dimension in the axial direction in the other group of the plurality of segments 11 disposed on the proximal end side with respect to the former group.

In this manner, the flexibility of a portion on the distal end side of the main body portion 10 can be increased, and the bending rigidity of a portion on the proximal end side can be increased.

As shown in FIG. 1B, the guide wire 1 in the present embodiment includes at least one second linear member (operation wire 40). The operation wire 40 is used for a bending operation. In the present embodiment, the guide wire 1 includes two operation wires 40. As shown in FIG. 2B, the two operation wires 40 are disposed to face each other at 180 degrees across the through-hole 114. The guide wire 1 may include one, three, or more operation wires 40.

As shown in FIG. 1B, one end of the operation wire 40 is coupled to the segment 11 disposed in the distal end portion of the main body portion 10. In the present embodiment, the operation wire 40 is coupled to the connection segment 11a. Instead of the present embodiment, the operation wire 40 may be coupled to the segment 11 disposed on the proximal end side with respect to the connection segment 11a. The operation wire 40 and the segment 11 may be coupled by any method such as embedding, adhesion, brazing, welding, or other methods. The other end of the operation wire 40 is coupled to the operation portion 30.

As shown in FIG. 2A or FIG. 2B, the operation wire 40 is inserted into an insertion hole 113 in the segment 11. The insertion hole 113 is provided in an outer edge portion of the segment 11 when viewed in the longitudinal direction. Specifically, the insertion hole 113 is formed within the wall thickness of the segment 11. Since one or a plurality of the operation wires 40 are pulled to the proximal end side, a bending operation can be performed on the main body portion 10 in a desired direction and at a desired angle.

As shown in FIG. 2B, the insertion hole 113 and the uneven engagement portion (recess portion 111 or protruding portion 112) are disposed to be shifted in the circumferential direction of the main body portion 10 when viewed in the axial direction. Preferably, the insertion hole 113 and the uneven engagement portion are disposed to be shifted by 90 degrees in the circumferential direction of the main body portion 10 when viewed in the axial direction. When a plurality of the insertion holes 113 are provided, a portion of the insertion holes 113 may be disposed to be shifted from the uneven engagement portion in the circumferential direction when viewed in the axial direction. For example, when four insertion holes 113 are disposed to be shifted from each other by 90 degrees, two insertion holes 113 may be disposed to be shifted from the uneven engagement portion when viewed in the axial direction. The remaining two insertion holes 113 may be disposed without being shifted from the uneven engagement portion in the circumferential direction when viewed in the axial direction. That is, the two remaining insertion holes 113 may be disposed to overlap the uneven engagement portion when viewed in a radial direction of the main body portion 10. Here, the radial direction of the main body portion 10 is a direction from an axis of the main body portion 10 toward a peripheral surface of the main body portion 10. Hereinafter, in some cases, the radial direction of the main body portion 10 may be simply referred to as the radial direction.

The main body portion 10 can be bent in an alignment direction of the operation wires 40 (up-down direction in FIG. 1B) by the two operation wires 40 disposed to face each other by 180 degrees. In this manner, the bending of the main body portion 10 can be operated while the bending angle between the adjacent segments 11 can be adjusted by the uneven engagement portion within a desired range as described above.

Instead of the present embodiment, the guide wire 1 may not include the operation wire 40. In addition, the guide wire 1 may have the operation wire 40, and may be formed to overlap the operation wire 40 and the uneven engagement portion in the radial direction.

The present invention is not limited to the above-described embodiment, and various modifications, improvements, and the like are also included as long as the object of the present invention is achieved.

The following modification examples can be appropriately combined.

In the present embodiment, the segment 11 includes the uneven engagement portion, but the present invention is not limited thereto. The segments 11 may not include the uneven engagement portion, and the adjacent segments 11 may not engage with each other.

In the present embodiment, the tension wire 20 penetrates the center of the segment 11 when viewed in the axial direction of the segment 11, but the present invention is not limited thereto. For example, the tension wire 20 may penetrate a peripheral edge portion of the segment 11 when viewed in the axial direction of the segment 11. The tension wire 20 may penetrate the inside of the wall thickness of the peripheral wall portion of the segment 11. Alternatively, the tension wire 20 may be disposed along an outer surface of the segment 11 (main body portion 10).

A shape of the uneven engagement portion is not limited to the above-described shape.

For example, as shown in FIG. 5A, the protruding portion 112 and the corresponding recess portion 111 may overlap in the radial direction. Specifically, the protruding portion 112 may have a cylindrical shape that protrudes in the axial direction. In addition, the recess portion 111 may be a portion common to a portion of the through-hole 114 (refer to FIG. 2A) on the proximal end side. Even in this recess portion 111 and this protruding portion 112, it is preferable that the width dimension is small in either direction in the axial direction. The width of the recess portion 111 or the protruding portion 112 in this case is a dimension in any direction orthogonal to the axial direction. In addition, it is preferable that the width of the protruding portion 112 or the recess portion 111 in the segment 11 on the distal end side is larger than the width of the protruding portion 112 or the recess portion 111 in the segment 11 on the proximal end side. In addition, it is preferable that the protruding dimension of the protruding portion 112 or the depth dimension of the recess portion 111 in the segment 11 on the distal end side is smaller than the protruding dimension of the protruding portion 112 or the depth dimension of the recess portion 111 in the segment 11 on the proximal end side.

In addition, as shown in FIG. 5B, the uneven engagement portions facing each other at 180 degrees may be formed to protrude or to be recessed in a direction opposite to the axial direction. Since the uneven engagement portion is formed in this way, the flexibility of the main body portion 10 in a desired direction can be increased. Specifically, the distal end of the main body portion 10 in FIG. 5B is easily bent upward on the paper surface.

In the present embodiment, the guide wire 1 includes only one tension wire 20, but the present invention is not limited thereto. The guide wire 1 may include two or more tension wires 20. For example, one tension wire 20 (distal end side tension wire 20) may be connected to the segment 11 in a most distal end as in the present embodiment, and the other tension wire 20 (proximal end side tension wire 20) may be connected to the segment 11 in an intermediate portion in the axial direction. It is preferable that the tension of the distal end side tension wire 20 and the tension of the proximal end side tension wire 20 can be separately adjusted by the operation portion 30. In this manner, the flexibility of the partial length region and the other partial length region in the flexible portion 12 can be individually adjusted. Specifically, the flexibility of the whole flexible portion 12 can be increased by the distal end side tension wire 20, and thereafter, the bending rigidity of the partial length region of the proximal end side flexible portion 12 can be increased by the proximal end side tension wire 20.

### <Second Embodiment>

### (Catheter)

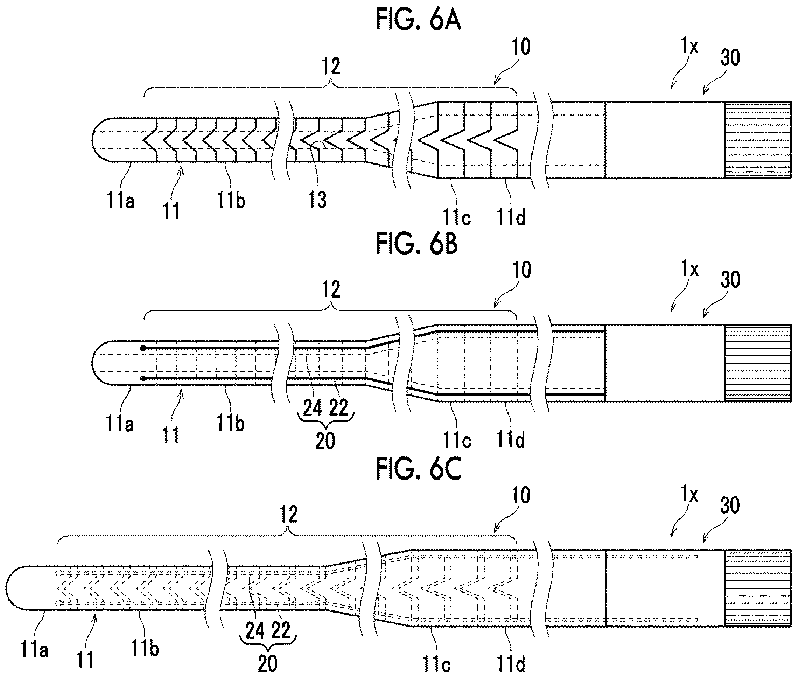

An outline of a catheter 1x according to the present embodiment will be described. FIGS. 6A to 6C are schematic views when the catheter 1x of the present embodiment is viewed from a side surface.

As shown in FIG. 6A, the catheter 1x includes the main body portion 10 and the operation portion 30. In the drawing, an outer layer 50 (to be described later) is omitted. The main body portion 10 is a member elongated and inserted into the body lumen, and includes a lumen 13. In FIG. 6A, the lumen 13 is shown by a dotted line. The operation portion 30 is formed in the proximal end of the main body portion 10. As shown in FIG. 6B, the main body portion 10 includes the plurality of segments 11 and a linear member 20. The plurality of segments 11 are continuously provided in the longitudinal direction of the main body portion 10, and can be separated from or closer to each other in the longitudinal direction. The linear member 20 is inserted into at least a portion of the plurality of segments 11. One end of the linear member 20 is connected to the operation portion 30, and the other end is connected to the connection segment 11a which is one of the segments 11. Since the tension of the linear member 20 extending between the connection segment 11a and the operation portion 30 is increased, the distance between the two segments 11 disposed adjacent to each other on the proximal end side with respect to the connection segment 11a is decreased, or the pressure contact force applied to the two segments 11 from each other is increased.

Details of the catheter 1x will be described.

The catheter 1x is an instrument inserted into the body lumen such as the blood vessel and injecting a liquid such as a drug into a desired location in the body lumen, and includes a so-called cannula. The lumen 13 passing through the center of the catheter 1x and extending in the axial direction of the catheter 1x is formed inside the catheter 1x. The lumen 13 is open in the distal end of the catheter 1x. The drug is injected into the desired location through the lumen 13.

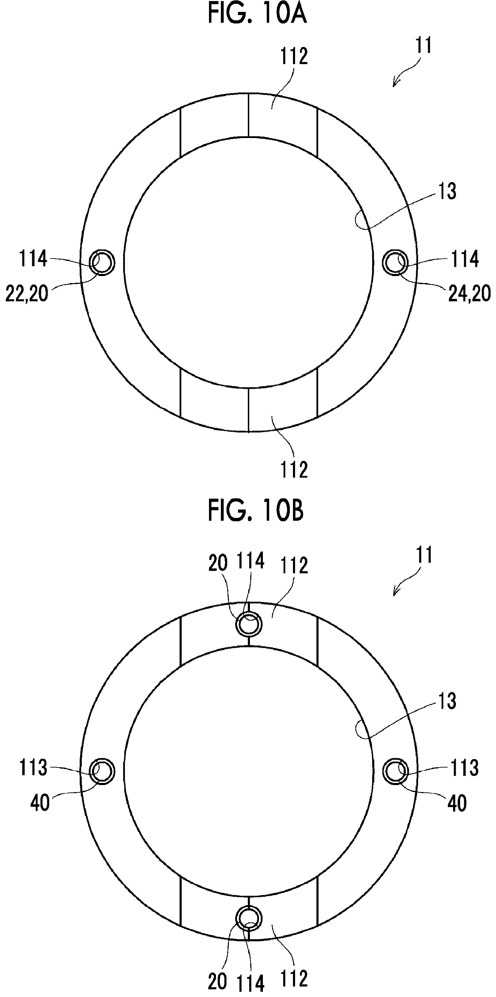

In the present embodiment, as shown in FIG. 10A, the wall portion of the segment 11 is provided with the through-hole 114 extending in the axial direction. The through-holes 114 are respectively open on end surface of the segment 11 in the axial direction. The tension wire 20 is inserted into the through-hole 114. In the present embodiment, the two through-holes 114 are provided to be aligned across the lumen 13 in a direction (left-right direction in the drawing) orthogonal to a direction (up-down direction in the drawing) in which the pair of recess portions 111 or the pair of protruding portions 112 face each other. The first tension wire 22 and the second tension wire 24 are respectively inserted into the two through-holes 114.

Instead of the present embodiment, the two through-holes 114 may be formed to be aligned across the lumen 13 in a direction (up-down direction in the drawing) in which the pair of recess portions 111 or the pair of protruding portions 112 face each other.

In addition, instead of the present embodiment, only one tension wire 20 may be inserted into the segment 11, or a plurality of the tension wires 20 including three or more may be inserted into the segment 11.

In the present embodiment, the tension of the two or more tension wires 20 is adjusted at the same time by one operation portion 30. In this manner, the tension of the plurality of tension wires 20 can be collectively operated, and the catheter 1x is less likely to be unexpectedly bent since the tension of one tension wire 20 unexpectedly decreases or increases.

Instead of this configuration, the two or more tension wires 20 may be respectively connected to the plurality of operation portions 30, and the tension may be individually adjusted by each of the plurality of operation portions 30. In this manner, the tension wire 20 can be used as the operation wire 40 (to be described later). For example, the distal end portion of the catheter 1x can be bent in a predetermined direction in such a manner that the first tension wire 22 is strongly pulled and the second tension wire 24 is weakly pulled or is not pulled.

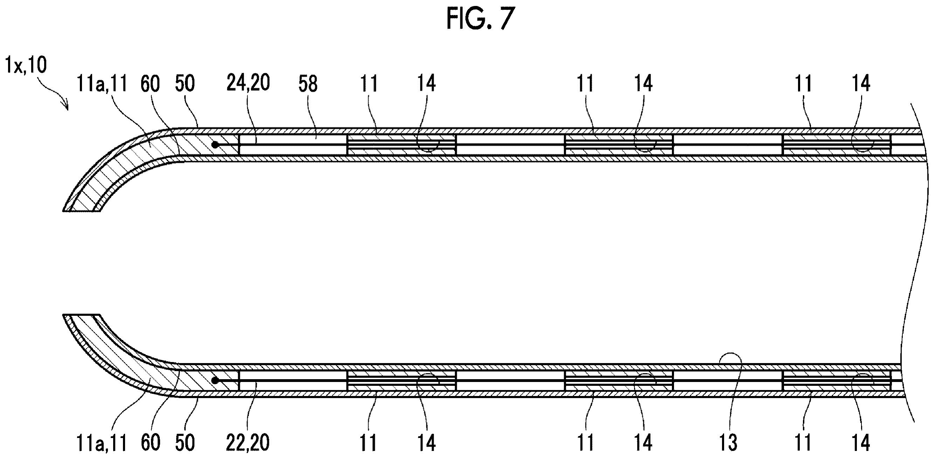

As shown in FIG. 7, the catheter 1x in the present embodiment is covered with an outer layer 50 and an inner layer 60. It is preferable that the outer layer 50 and the inner layer 60 are formed of resin materials. The outer layer 50 may be formed of the same resin material as the inner layer 60, or may be formed of a different resin material. It is preferable that the outer layer 50 is formed of engineering plastic such as nylon-based plastic, urethane-based plastic, or other plastic having excellent strength, or an elastomer having similar properties such as strength. It is preferable that the inner layer 60 is formed of PTFE.

In the present embodiment, the outer layer 50 is an outermost layer of the catheter 1x. The outer layer 50 covers at least the outer diameter side of the segment 11 in the flexible portion 12. In addition, the outer layer 50 is disposed to extend between two adjacent segments 11. In the present embodiment, the inner layer 60 is an innermost layer of the catheter 1x. The inner layer 60 covers the inner diameter side of the segment 11 in the flexible portion 12, and is disposed to extend between two adjacent segments 11.

In the present embodiment, each of the outer layer 50 and the inner layer 60 is fixed to the connection segment 11a by fusion welding, adhesion, or the like. On the other hand, in the other segments 11 disposed on the proximal end side with respect to the connection segment 11a, each of the outer layer 50 and the inner layer 60 may not be fixed. In other words, it is preferable that the segment 11 disposed on the proximal end side with respect to the connection segment 11a can slide on the outer layer 50 or the inner layer 60. Instead of this configuration, the outer layer 50 and the inner layer 60 may be respectively fixed to the other segment 11 disposed on the proximal end side with respect to the connection segment 11a. In this case, as will be described later, the inner layer 60 and the outer layer 50 may be formed of a resin material that is expandable and contractible in the axial direction (resin material having stretchability in the axial direction). Since the outer layer 50 and the inner layer 60 are expandable and contractible in the axial direction, as will be described later, the outer layer 50 and the inner layer 60 are stretched or compressed before and after the tension wire 20 is pulled toward the proximal end side. In this manner, the dimension of the outer layer 50 or the inner layer 60 in the axial direction can be changed. As a result, the outer layer 50 and the inner layer 60 are less likely to be folded in a bellows shape when the tension wire 20 is pulled to the proximal end side. Therefore, it is possible to suppress a possibility that a portion of the lumen 13 is blocked by the outer layer 50 and the inner layer 60 which are folded in the bellows shape or a possibility that the outer diameter of the catheter 1x is increased by the outer layer 50 and the inner layer 60 which are folded in the bellows shape.

When the tension wire 20 is pulled by the operation portion 30 to apply the tension to the tension wire 20, the connection segment 11a connected to the distal end of the tension wire 20 is pulled to the proximal end side.

When each of the outer layer 50 and the inner layer 60 is not fixed to the other segment 11 disposed on the proximal end side with respect to the connection segment 11a, the segment 11 can relatively slide in the axial direction on the outer layer 50 and the inner layer 60. Therefore, when the connection segment 11a is pulled to the proximal end side, the outer layer 50 or the inner layer 60 slides toward the proximal end on a peripheral surface or an inner peripheral surface of the segment 11 adjacent to the proximal end side of the connection segment 11a, and the connection segment 11a and the segment 11 adjacent to the proximal end side of the connection segment 11a can be closer to each other. Furthermore, an end surface of the connection segment 11a and an end surface of the segment 11 adjacent to the proximal end side of the connection segment 11a can come into contact with each other, and can be further in pressure contact with each other. In this way, as the connection segment 11a is pulled to the proximal end side, the segment 11 slides in a space interposed between the outer layer 50 and the inner layer 60. Therefore, the distance between the segments 11 can be closer, and the pressure contact force between the segments 11 can be increased.

In this case, one segment 11 may come into contact with the adjacent segment 11, and may be directly in pressure contact with the adjacent segment 11, or may be indirectly in pressure contact with the adjacent segment 11 with a portion of the outer layer 50 or the inner layer 60 interposed between the one segment 11 and the adjacent segment 11.

On the contrary, when the tension wire 20 is loosened by the operation portion 30, the segments 11 slide in the space interposed between the outer layer 50 and the inner layer 60. In this manner, the pressure contact force between the adjacent segments 11 decreases.

When each of the outer layer 50 and the inner layer 60 is fixed to each of the segments on the proximal end side with respect to the connection segment 11a, and when the segments 11 are closer to each other, the outer layer 50 and the inner layer 60 are compressed in the axial direction, and when the segments 11 are separated from each other, the outer layer 50 and the inner layer 60 are stretched in the axial direction. For example, when the tension wire 20 is pulled by operating the operation portion 30, the connection segment 11a is pulled to the proximal end side, and the connection segment 11a moves closer to the segment 11 adjacent to the proximal end side of the connection segment 11a. In this case, since the outer layer 50 and the inner layer 60 are fixed to the segment 11 and the connection segment 11a, the outer layer 50 and the inner layer 60 which extend between the segment 11 and the connection segment 11a are compressed in the axial direction when a distance between the outer layer 50 and the inner layer 60 decreases. Specifically, the dimension of the outer layer 50 or the inner layer 60 in the axial direction is decreased in such a manner that the outer layer 50 or the inner layer 60 is folded in a bellows shape or the like. Alternatively, the dimensions of the outer layer 50 and the inner layer 60 in the axial direction may be decreased by maintaining a state where the outer layer 50 and the inner layer 60 are compressed in the axial direction without folding the outer layer 50 or the inner layer 60. In this case, it is preferable that the outer layer 50 or the inner layer 60 has a property (pressure resistance) capable of withstanding the compression when the outer layer 50 or the inner layer 60 is compressed in the axial direction, and has a property of being easily deformed by the compression to change the dimension in the axial direction. Alternatively, as a result that the outer layer 50 and the inner layer 60 having stretchability in the axial direction are compressed in the axial direction, the tension of the inner layer 60 and the outer layer 50 may be relaxed. Specifically, in a state before the tension wire 20 is pulled to the proximal end side by the operation portion 30, the outer layer 50 and the inner layer 60 are stretched in the axial direction, and when the tension wire 20 is pulled and the outer layer 50 and the inner layer 60 are compressed in the axial direction, the tension applied to the outer layer 50 and the inner layer 60 may be relaxed, and the dimension of the outer layer 50 and the inner layer 60 in the axial direction may decrease.

In this case, one segment 11 may come into contact with the adjacent segment 11, and may be directly in pressure contact with the adjacent segment 11. In addition, the one segment 11 may be indirectly in pressure contact with the adjacent segment 11 with a portion of the outer layer 50 or the inner layer 60 interposed between the one segment 11 and the adjacent segment 11.

On the contrary, when the tension of the tension wire 20 is loosened by operating the operation portion 30, the distance between the segments 11 in the axial direction increases. Therefore, the above-described bellows shape or the like is eliminated, and the dimension of the outer layer 50 or the inner layer 60 extending between the segments 11 in the axial direction increases.

In the present embodiment, between the two adjacent segments 11, a substantially entire space (space defined by the outer layer 50, the inner layer 60, and end surfaces of the two adjacent segments 11) between the outer layer 50 and the inner layer 60 is a hollow portion 58. The tension wire 20 passes through the hollow portion 58 in the axial direction.

Instead of the present embodiment, a space between the two segments 11 may be filled with a member such as a resin. Between two adjacent segments 11, a space between the outer layer 50 and the inner layer 60 may be formed to be substantially solid by using a member such as the resin. In this manner, rigidity of the flexible portion 12 can be set to a desired value or greater.

For example, a tubular resin member having the same shape as the segment 11 may be disposed. The resin member is provided with a hole that penetrates in the axial direction, and the tension wire 20 is inserted into the hole. It is preferable that the resin member is formed of a material having a smaller elastic modulus (Young's modulus) than the segment 11. Since the resin member is soft, the flexible portion 12 can have prescribed flexibility. When the tension of the tension wire 20 increases, one segment 11 applies the pressure contact force to the adjacent segment 11 via the resin member. The resin member and the segment 11 may adhere to each other, or may not adhere to each other.

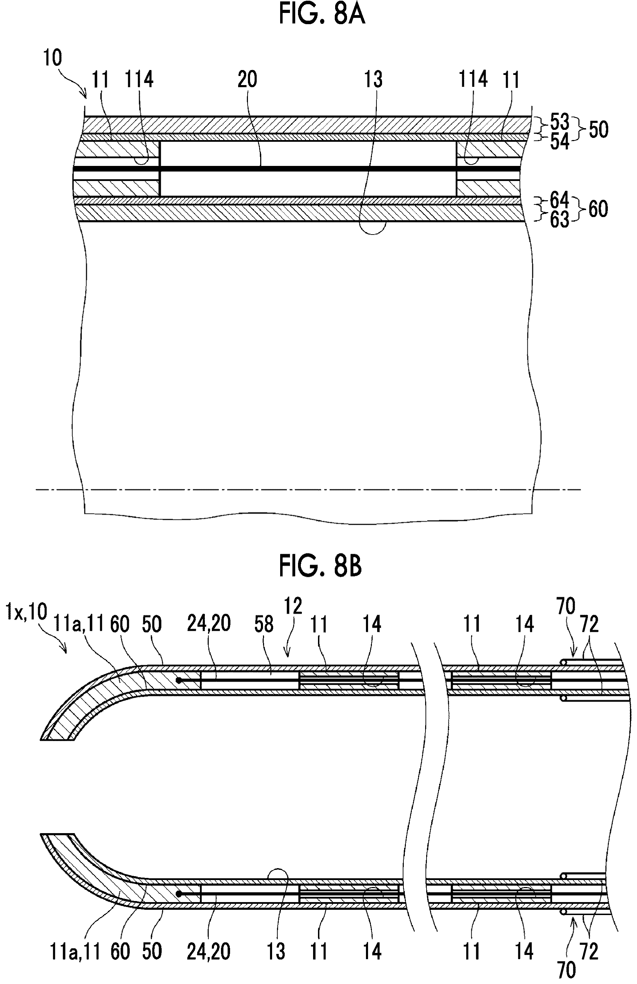

As described above, the catheter 1x further includes the outer layer 50 that covers the outer diameter side of each segment 11 and the inner layer 60 that covers the inner diameter side of each segment 11. In addition, the outer layer 50 and the inner layer 60 may not be fixed to the segment 11, and may be relatively slidable on the surface of the segment 11 in the axial direction. Here, the description that the outer layer 50 and the inner layer 60 are not fixed to the segment 11 particularly means that the outer layer 50 and the inner layer 60 are not fixed to the segment on the proximal end side with respect to the connection segment 11a. The outer layer 50 and the inner layer 60 may be fixed to the connection segment 11a. In this case, as shown in FIG. 8A, each of the outer layer 50 and the inner layer 60 includes first layers 53 and 63 and second layers 54 and 64. It is preferable that the second layers 54 and 64 can come into contact with the segment 11. In addition, it is preferable that the segments 11 can more smoothly slide on the second layers 54 and 64 than on the first layers 53 and 63.

The first layers 53 and 63 and the second layers 54 and 64 extend in the axial direction and the circumferential direction of the segment 11. The first layers 53 and 63 are layers disposed outside the second layers 54 and 64 in a direction (referred to as a facing direction) in which the inner layer 60 and the outer layer 50 face each other. The facing direction coincides with the up-down direction in FIG. 8A. The inner side in the facing direction is a direction toward a space interposed between the outer layer 50 and the inner layer 60, and the outer side in the facing direction is a direction opposite to the inner side. That is, the second layers 54 and 64 are disposed to be capable of coming into contact with the segment 11, and the first layers 53 and 63 are separated from the segments 11 across the second layers 54 and 64. In other words, the first layer 53 in the outer layer 50 is disposed outside the second layer 54 in the outer layer 50 in the radial direction of the catheter 1x. On the other hand, the first layer 63 in the inner layer 60 is disposed inside the second layer 64 in the inner layer 60 in the radial direction of the catheter 1x.

Since the segment 11 can more smoothly slide on the second layers 54 and 64 than on the first layers 53 and 63, the segment 11 is less likely to be caught in the outer layer 50 or the inner layer 60 when the segment 11 moves in the space between the outer layer 50 and the inner layer 60.

The description that the second layers 54 and 64 can come into contact with the segment 11 means that the second layers 54 and 64 may come into contact with the segment 11 at any timing when the segment 11 is moved in the axial direction by the operation of the operation portion 30.

The description that the segment 11 can more smoothly slide on the second layers 54 and 64 than on the first layers 53 and 63 means that a force required for pushing and moving the segment 11 placed on the second layers 54 and 64 by a predetermined distance is smaller than a force required for pushing and moving the segment 11 placed on the first layers 53 and 63 by a predetermined distance.

For example, a configuration that the segment 11 can more smoothly slide on the second layers 54 and 64 than the first layers 53 and 63 may be realized by forming the second layers 54 and 64 of a material having a smaller friction coefficient than a material forming the first layers 53 and 63. For example, the second layers 54 and 64 may be formed of the material different from that of the first layers 53 and 63, or surface roughness of the second layers 54 and 64 may be larger or smaller than surface roughness of the first layers 53 and 63. In this manner, a difference in the friction coefficient may be generated. In addition, the second layers 54 and 64 may be formed of a mesh material or the like. In this manner, the segment 11 may more smoothly slide on the second layers 54 and 64 than on the first layers 53 and 63.

When the outer layer 50 and the inner layer 60 are not fixed to the segment 11 and are relatively slidable on the surface of the segment 11 in the axial direction, the catheter 1x may include a pulling mechanism 70 for pulling the outer layer 50 and the inner layer 60 to the proximal end side. When the tension of the tension wire 20 is increased, the outer layer 50 and the inner layer 60 are pulled to the proximal end side by the pulling mechanism 70. Therefore, the outer layer 50 or the inner layer 60 is less likely to be irregularly folded between two adjacent segments 11. In this manner, it is possible to suppress a possibility that a portion of the lumen 13 is unexpectedly blocked by the outer layer 50 or the inner layer 60 that is irregularly folded, or a possibility that the outer diameter of the catheter 1x is unexpectedly increased.

The pulling mechanism 70 is a mechanism that can pull the outer layer 50 and the inner layer 60 to the proximal end side by the operation in the operation portion 30 (refer to FIG. 6A). For example, as shown in FIG. 8B, one end of the pulling wire 72 is connected to a portion of the outer layer 50 and a portion of the inner layer 60 which are disposed on the proximal end side with respect to the flexible portion 12, and the other end of the pulling wire 72 is connected to the operation portion 30 (refer to FIG. 6A). When the proximal end portions of the outer layer 50 and the inner layer 60 are pulled to the proximal end side by operating the operation portion 30, the outer layer 50 and the inner layer 60 can slide to the proximal end side as a whole.

The outer layer 50 and the inner layer 60 may be more regularly and reproducibly folded when two adjacent segments 11 move closer to each other. When the outer layer 50 and the inner layer 60 are compressed in the axial direction to have an irregular shape, the lumen 13 may be blocked or the outer shape of the catheter 1x may become larger. However, occurrence of the event is suppressed in such a manner that the outer layer 50 or the inner layer 60 is regularly and reproducibly folded as described above.

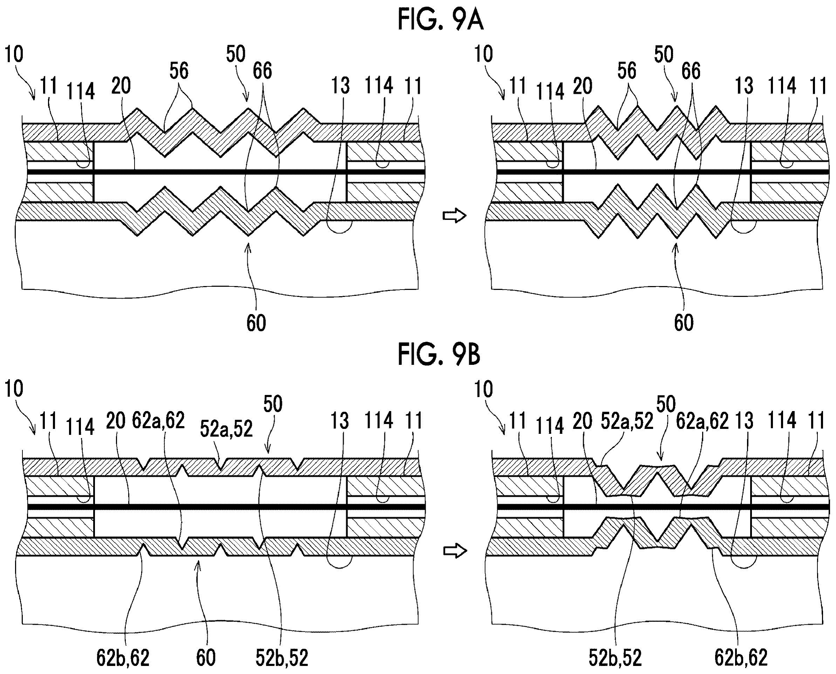

For example, as shown in FIG. 9A, the outer layer 50 or the inner layer 60 may include fold lines 56 and 66 along the circumferential direction of the segment 11. More specifically, the outer layer 50 and the inner layer 60 may be formed to have the fold lines 56 and 66 in a natural state. In the fold line in FIG. 9A, the outer layer 50 or the inner layer 60 has a bellows shape by repeating mountain folding or valley folding of the outer layer 50 or the inner layer 60 in the axial direction. In this manner, even when the tension wire 20 shown on a left side in FIG. 9A is in a state shown on a right side in FIG. 9A in which the tension is applied, the outer layer 50 and the inner layer 60 can be folded in the bellows shape in which the mountain folding and the valley folding are repeated.

Alternatively, as shown in FIG. 9B, slits 52 and 62 may be provided in the outer layer 50 or the inner layer 60. The slits 52 and 62 extend in the circumferential direction of the segment 11 (catheter 1x). In FIG. 9B, slits (outer slits 52a and 62a and inner slits 52b and 62b) are provided on each of both surfaces of the outer layer 50. When the tension is applied to the tension wire 20 shown on the left side in FIG. 9B and the two adjacent segments 11 move closer to each other, as shown on the right side in FIG. 9B, each of the slits 52 and 62 is opened, and the outer layer 50 or the inner layer 60 is likely to be bent while the slits 52 and 62 are used as starting points. In this manner, the outer layer 50 or the inner layer 60 can be regularly folded.

The bending rigidity of a portion on the proximal end side in the outer layer 50 or the inner layer 60 covering the flexible portion 12 may be greater than the bending rigidity of a portion on the distal end side in the outer layer 50 or the inner layer 60 covering the flexible portion 12. In this manner, the flexibility toward the distal end portion in the flexible portion 12 can be increased, and the bending rigidity toward the proximal end portion in the flexible portion 12 can be increased. In this stage, a portion on the proximal end side or a portion on the distal end side of the outer layer 50 or the inner layer 60 covering the flexible portion 12 will be referred to as a proximal end portion or a distal end portion of the outer layer 50 or the inner layer 60.

For example, the thickness of the proximal end portion of the outer layer 50 or the inner layer 60 may be larger than the thickness of the distal end portion of the outer layer 50 or the inner layer 60. Alternatively, as shown in FIG. 9A, when the above-described fold lines 56 and 66 are provided, a separation distance between the adjacent fold lines 56 and 66 in the axial direction in the proximal end portion of the outer layer 50 or the inner layer 60 may be larger than the separation distance in the distal end portion of the outer layer 50 or the inner layer 60. As shown in FIG. 9B, when the slits 52 and 62 are provided, a separation distance between the adjacent slits 52 and 62 in the axial direction in the proximal end portion of the outer layer 50 or the inner layer 60 may be larger than the distance in the distal end portion of the outer layer 50 or the inner layer 60.

Alternatively, the stretchability or the pressure resistance of the inner layer 60 or the outer layer 50 may be changed such that the distance between the segments 11 on the distal end side is smaller than the distance between the segments 11 on the proximal end side when the tension wire 20 is pulled to the proximal end side. Specifically, when the segment 11 is fixed to the inner layer 60 and the outer layer 50 as described above and the inner layer 60 and the outer layer 50 have the stretchability in the axial direction, the inner layer 60 and the outer layer 50 on the distal end side may have large stretchability, and the inner layer 60 and the outer layer 50 on the proximal end side may have stretchability smaller than the stretchability of the inner layer 60 and the outer layer 50 on the distal end side. Alternatively, when the segment 11 is fixed to the inner layer 60 and the outer layer 50 as described above, and the inner layer 60 and the outer layer 50 have a property of being easily deformable by the compression in the axial direction, the amount of deformation of the inner layer 60 and the outer layer on the distal end side in the axial direction when the equal compression is applied in the axial direction may be larger than the amount of deformation of the inner layer 60 and the outer layer on the proximal end side in the axial direction. In this case, when the equal compression is applied in the axial direction, the dimensions of the inner layer 60 and the outer layer 50 on the distal end side become smaller than the dimensions of the inner layer 60 and the outer layer 50 on the proximal end side in the axial direction.

As shown in FIG. 10B, the catheter 1x of the present embodiment may include the operation wire 40. In this case, as in the first embodiment, the operation wire 40 is inserted into the insertion hole 113 provided in the segment 11. It is preferable that the insertion hole 113 and the uneven engagement portion are disposed to be shifted in the circumferential direction of the main body portion when viewed in the longitudinal direction of the catheter 1x. More specifically, in the present embodiment, the pair of uneven engagement portions (protruding portions 112) face each other in the up-down direction in the drawing. It is preferable that the insertion holes 113 are disposed to be aligned across the lumen 13 in a direction (in the drawing, the left-right direction) orthogonal to a direction (in the drawing, the up-down direction) in which the pair of uneven engagement portions face each other. In addition, in this case, the through-hole 114 into which the tension wire 20 is inserted may be formed to pass through the uneven engagement portion (protruding portion 112).

The catheter 1x in the present embodiment may include the uneven engagement portion, as in the guide wire 1 in the first embodiment. More specifically, in the present embodiment, one segment 11 and the other segment 11 engage with each other such that the protruding portion 112 and the recess portion 111 overlap each other when viewed in the circumferential direction of the main body portion 10.

In addition, the width of the protruding portion 112 and the width of the recess portion 111 are narrowed toward the distal end of the main body portion 10.

The width of the protruding portion 112 or the recess portion 111 provided in the first segment 11b is larger than the width of the protruding portion 112 or the recess portion 111 provided in the second segment 11c.

In addition, the dimension of the protruding portion 112 or the recess portion 111 of the first segment 11b in the longitudinal direction of the catheter 1x is smaller than the dimension of the protruding portion 112 or the recess portion 111 of the second segment 11c in the longitudinal direction of the catheter 1x.

Furthermore, the dimension of the first segment 11b in the longitudinal direction of the catheter 1x is smaller than the dimension of the first segment 11b in the width direction.

In addition, the dimension of the first segment 11b in the longitudinal direction of the catheter 1x is smaller than the dimension of the second segment 11c in the longitudinal direction.

According to this configuration, a portion of the flexible portion on the distal end side can have higher flexibility than a portion of the flexible portion on the proximal end side.

The embodiment includes the following technical ideas.
(1) There is provided a guide wire including a main body portion elongated and inserted into a body lumen, and an operation portion formed in a proximal end of the main body portion. The main body portion includes a plurality of segments continuously provided in a longitudinal direction of the main body portion and configured to be separated from or closer to each other in the longitudinal direction, and a linear member inserted into at least a portion of the plurality of segments. The linear member is connected to the operation portion in one end, and is connected to a connection segment which is one of the segments in the other end. A distance between two of the segments disposed on a proximal end side with respect to the connection segment and adjacent to each other is decreased or a pressure contact force applied to the two segments from each other is increased by increasing tension of the linear member extending between the connection segment and the operation portion.
(2) In the guide wire according to (1), one of the segments has an uneven engagement portion that engages with an uneven engagement portion of the other adjacent segment.
(3) In the guide wire according to (2), the one segment has a recess portion as the uneven engagement portion in a proximal end portion. The other segment adjacent to the one segment on a proximal end side of the one segment has a protruding portion as the uneven engagement portion in a distal end portion. The one segment and the other segment engage with each other such that the protruding portion and the recess portion overlap each other in a circumferential direction of the main body portion.
(4) In the guide wire according to (3), a width of the protruding portion and a width of the recess portion are narrowed toward a distal end of the main body portion.
(5) In the guide wire according to (4), the width of the protruding portion or the width of the recess portion provided in a first segment is larger than the width of the protruding portion or the width of the recess portion provided in a second segment disposed on the proximal end side with respect to the first segment.
(6) In the guide wire according to (4) or (5), a dimension of the protruding portion or the recess portion of a first segment in the longitudinal direction is smaller than a dimension of the protruding portion or the recess portion of a second segment disposed on the proximal end side with respect to the first segment, which is a dimension in the longitudinal direction.
(7) In the guide wire according to (6), a dimension of the first segment in the longitudinal direction is smaller than a dimension of the first segment in a direction orthogonal to the longitudinal direction.
(8) In the guide wire according to (7), the dimension of the first segment in the longitudinal direction is smaller than the dimension of the second segment in the longitudinal direction.
(9) The guide wire according to any one of (2) to (5) further includes at least one second linear member for a bending operation, one end of which is coupled to the segment disposed in a distal end portion of the main body portion, and the other end of which is coupled to the operation portion. The second linear member is inserted into an insertion hole provided in an outer edge portion of the segment when viewed in the longitudinal direction. The insertion hole and the uneven engagement portion are disposed to be shifted from each other in a circumferential direction of the main body portion when viewed in the longitudinal direction.
(10) There is provided a catheter including a main body portion elongated, inserted into a body lumen, and having a lumen, and an operation portion formed in a proximal end of the main body portion. The main body portion includes a plurality of segments continuously provided in a longitudinal direction of the main body portion and configured to be separated from or closer to each other in the longitudinal direction, and a linear member inserted into at least a portion of the plurality of segments. The linear member is connected to the operation portion in one end, and is connected to a connection segment which is one of the segments in the other end. A distance between two of the segments disposed on a proximal end side with respect to the connection segment and adjacent to each other is decreased or a pressure contact force applied to the two segments from each other is increased by increasing tension of the linear member extending between the connection segment and the operation portion.
(11) The catheter according to (10) further includes an outer layer that covers an outer diameter side of each segment and an inner layer that covers an inner diameter side of each segment. The outer layer and the inner layer are not fixed to the segment, and are relatively slidable on a surface of the segment in an axial direction. Each of the outer layer and the inner layer includes a first layer and a second layer configured to come into contact with the segment, on which the segment is more smoothly slidable than on the first layer.
(12) In the catheter according to (10), the catheter further includes an outer layer that covers an outer diameter side of each segment and an inner layer that covers an inner diameter side of each segment. The outer layer and the inner layer have a fold line along a circumferential direction of the segment.

### Reference Signs List

- 1:: guide wire
- 1x:: catheter
- 10:: main body portion
- 11:: segment
- 11a:: connection segment
- 11b:: first segment
- 11c:: second segment
- 11d:: proximal end segment
- 11e:: adjacent segment
- 111:: recess portion
- 111a:: recess portion wall portion
- 112:: protruding portion
- 112a:: protruding wall portion
- 113:: insertion hole
- 114:: through-hole
- 12:: flexible portion
- 13:: lumen
- 20:: tension wire, distal end side tension wire, proximal end side tension wire, linear member
- 21:: crossing portion
- 22:: first tension wire
- 24:: second tension wire
- 30:: operation portion
- 31:: twisted portion
- 31a:: male screw
- 32:: female screw portion
- 32a:: female screw
- 40:: operation wire, second linear member
- 50:: outer layer
- 52:: slit
- 52a:: outer slit
- 52b:: inner slit
- 53:: first layer
- 54:: second layer
- 56:: fold line
- 58:: hollow portion
- 60:: inner layer
- 62:: slit
- 62a:: outer slit
- 62b:: inner slit
- 63:: first layer
- 64:: second layer
- 66:: fold line
- 70:: pulling mechanism
- 72:: pulling wire

## Claims

1. A guide wire comprising:
a main body portion elongated and inserted into a body lumen; and
an operation portion formed in a proximal end of the main body portion,
wherein the main body portion includes a plurality of segments continuously provided in a longitudinal direction of the main body portion and configured to be separated from or closer to each other in the longitudinal direction, and a linear member inserted into at least a portion of the plurality of segments,
the linear member is connected to the operation portion in one end, and is connected to a connection segment which is one of the segments in the other end, and
a distance between two of the segments disposed on a proximal end side with respect to the connection segment and adjacent to each other is decreased or a pressure contact force applied to the two segments from each other is increased by increasing tension of the linear member extending between the connection segment and the operation portion.

2. The guide wire according to claim 1,
wherein one of the segments has an uneven engagement portion that engages with an uneven engagement portion of the other adjacent segment.

3. The guide wire according to claim 2,
wherein the one segment has a recess portion as the uneven engagement portion in a proximal end portion,
the other segment adjacent to the one segment on a proximal end side of the one segment has a protruding portion as the uneven engagement portion in a distal end portion, and
the one segment and the other segment engage with each other such that the protruding portion and the recess portion overlap each other in a circumferential direction of the main body portion.

4. The guide wire according to claim 3,
wherein a width of the protruding portion and a width of the recess portion are narrowed toward a distal end of the main body portion.

5. The guide wire according to claim 4,
wherein the width of the protruding portion or the width of the recess portion provided in a first segment is larger than the width of the protruding portion or the width of the recess portion provided in a second segment disposed on the proximal end side with respect to the first segment.

6. The guide wire according to claim 4 or 5,
wherein a dimension of the protruding portion or the recess portion of a first segment in the longitudinal direction is smaller than a dimension of the protruding portion or the recess portion of a second segment disposed on the proximal end side with respect to the first segment, which is a dimension in the longitudinal direction.

7. The guide wire according to claim 6,
wherein a dimension of the first segment in the longitudinal direction is smaller than a dimension of the first segment in a direction orthogonal to the longitudinal direction.

8. The guide wire according to claim 7,
wherein the dimension of the first segment in the longitudinal direction is smaller than the dimension of the second segment in the longitudinal direction.

9. The guide wire according to any one of claims 2 to 5, further comprising:
at least one second linear member for a bending operation, one end of which is coupled to the segment disposed in a distal end portion of the main body portion, and the other end of which is coupled to the operation portion,
wherein the second linear member is inserted into an insertion hole provided in an outer edge portion of the segment when viewed in the longitudinal direction, and
the insertion hole and the uneven engagement portion are disposed to be shifted from each other in a circumferential direction of the main body portion when viewed in the longitudinal direction.

10. A catheter comprising:
a main body portion elongated, inserted into a body lumen, and having a lumen; and
an operation portion formed in a proximal end of the main body portion,
wherein the main body portion includes a plurality of segments continuously provided in a longitudinal direction of the main body portion and configured to be separated from or closer to each other in the longitudinal direction, and a linear member inserted into at least a portion of the plurality of segments,
the linear member is connected to the operation portion in one end, and is connected to a connection segment which is one of the segments in the other end, and
a distance between two of the segments disposed on a proximal end side with respect to the connection segment and adjacent to each other is decreased or a pressure contact force applied to the two segments from each other is increased by increasing tension of the linear member extending between the connection segment and the operation portion.

11. The catheter according to claim 10, further comprising:
an outer layer that covers an outer diameter side of each segment; and
an inner layer that covers an inner diameter side of each segment,
wherein the outer layer and the inner layer are not fixed to the segment, and are relatively slidable on a surface of the segment in an axial direction, and
each of the outer layer and the inner layer includes a first layer and a second layer configured to come into contact with the segment, on which the segment is more smoothly slidable than on the first layer.

12. The catheter according to claim 10, further comprising:
an outer layer that covers an outer diameter side of each segment; and
an inner layer that covers an inner diameter side of each segment, and
wherein the outer layer and the inner layer have a fold line along a circumferential direction of the segment.
